# EUROPEAN PATENT APPLICATION

(11) **EP 1 095 959 A2**
(43) Date of publication of application: **02.05.2001**
(21) Application number: 00309298.8
(22) Date of filing: 23.10.2000
(51) Int. Cl.: C08G 77/42, C08B 15/05, A61K 7/00

(54) **Cosmetic composition**

(30) Priority: 28.10.1999 JP 30706999
(71) Applicant: SHIN-ETSU CHEMICAL CO., LTD., Chiyoda-ku Tokyo (JP)
(72) Inventor: Ono, Ichiro, Matsuida-machi, Usui-gun, Gunma-ken (JP); Yamamoto, Akira, Matsuida-machi, Usui-gun, Gunma-ken (JP)
(74) Representative: Vinsome, Rex Martin

(57) **Abstract**

Disclosed is a cosmetic composition, such as a cream and anti-perspirant composition, improved in respect of the sustainability of the effect of treatment therewith and excellent feeling of use imparted to the users. The most characteristic ingredient of the inventive cosmetic composition is a silicone-grafted polysaccharide compound having a specified acid value which is a reaction product of a carboxyl-containing polysaccharide compound such as hydroxypropyl methyl cellulose phthalate and a diorganopolysiloxane terminated at a single polymeric molecular chain end with a 2-(3,4-epoxycyclohexyl)ethyl group, preferably, leaving a controlled amount of the carboxyl groups by partial neutralization.

## Description

### BACKGROUND OF THE INVENTION

The present invention relates to a cosmetic composition which can be prepared in an economical process and capable of giving a quite pleasant feeling of use to the users of the cosmetic composition with sustainability of the effect of cosmetic treatment.

While it is known that a variety of cosmetic and toiletry compositions are formulated with a film-forming agent, examples of most widely employed film-forming agents include polymeric compounds such as acrylic resins, vinyl resins and polysaccharide compounds. In addition to these polymeric compounds, a class of polymers obtained by grafting an organopolysiloxane to the above mentioned polymeric compounds are highlighted and increasingly employed in recent years as a film-forming agent in cosmetic compositions by virtue of their excellent properties relative to water-repellency, lubricity, stability and so on. Among the film-forming agents of this class, in particular, the organopolysiloxane-grafted polysaccharide compounds, referred to as a silicone-grafted polysaccharide hereinafter, are the most promising in respect of the strength of the film formed therefrom and good adhesion to the surface of the human body treated with the cosmetic composition. A problem in the use of such a silicone-grafted polysaccharide is that the grafting amount of the organopolysiloxane moiety onto the polysaccharide compound is sometimes not high enough as desired because, different from synthetic polymers prepared by the polymerization reaction of a monomeric compound, the polysaccharide compound is a natural product or a semi-synthetic polymer without high reactivity with an organopolysiloxane compound.

Several methods are known for the grafting reaction of an organopolysiloxane to a polysaccharide compound as disclosed in Japanese Patent Publications 64-8001 and 64-11201, Japanese Patents 071051 and 071084 and Japanese Patent Kokai 7-70204 and 9-136901. The silicone-grafted polysaccharide products obtained by the reaction of these prior art methods, however, are not quite satisfactory as an ingredient in cosmetic compositions in respect of the low content of the grafting moiety of organopolysiloxane even by the use of a catalyst for promoting the grafting reaction as a consequence of the so vast difference in the chemical natures of an organopolysiloxane and a polysaccharide compound as the reactants pertaining to the grafting reaction. This is the principal reason for the relatively few number of formulations of cosmetic compositions in which a silicone-grafted polysaccharide is used as a film-forming agent. In addition, the product of the grafting reaction between an organopolysiloxane and a polysaccharide compound can be used as a film-forming agent in a cosmetic composition only after a complicated purification process to remove the unreacted reactants and the catalyst leading to expensiveness of the silicone-grafted polysaccharides in general.

### SUMMARY OF THE INVENTION

The present invention accordingly has an object, in view of the above described problems and disadvantages in the cosmetic compositions formulated with a conventional silicone-grafted polysaccharide as a film-forming agent, to provide a silicone-grafted polysaccharide of a novel type which can be prepared inexpensively by virtue of the high reactivity of the reactants even in the absence of any catalyst to leave little amounts of unreacted reactants unnecessitating the complicated and expensive purification process as well as to provide a cosmetic composition formulated with such a silicone-grafted polysaccharide capable of exhibiting excellent cosmetic dressing effect and giving a pleasant and comfortable feeling to the users of the cosmetic composition.

Thus, the silicone-grafted polysaccharide compound provided by the invention and used as an essential ingredient in the inventive cosmetic composition is a reaction product between: (A) a polysaccharide compound having at least one carboxyl group in a molecule and having solubility in an organic solvent, such as hydroxypropyl methyl cellulose phthalate; and (B) a diorganopolysiloxane represented by the general formula

E-CH₂CH₂-(-SiR₂-O-)ₙ-SiR₃, (I)

in which E is a 3,4-epoxycyclohexyl group, each R is, independently from the others, a monovalent hydrocarbon group having 1 to 10 carbon atoms and the subscript n is a positive integer in the range from 3 to 200, which has an acid value in the range from 10 to 250 mg KOH/g and of which the content of the organopolysiloxane moiety is in the range from 1 to 60% by weight.

The cosmetic composition provided by the present invention comprises, as a blend:
(a) from 0.5 to 30.0% by weight of the above defined silicone-grafter polysaccharide; and
(b) from 0.5 to 99.5% by weight of a compound having, in a molecule, at least one aliphatic hydroxyl group,
   the balance, if any, being compounds admissible in a cosmetic composition.

In addition to the above defined essential components (a) and (b), the inventive cosmetic composition is formulated with addition of (c) from 0.5 to 50.0% by weight of an oil compound.

### DETAILED DESCRIPTION OF THE PREFERRED EMBODIMENTS

The silicone-grafted polysaccharide used as the component (a) in the inventive cosmetic composition is a product obtained by the grafting reaction between a polysaccharide compound soluble in an organic solvent and having at least one carboxyl group in a molecule as the reactant (A) and a diorganopolysiloxane terminated at one of the molecular chain ends with a 2-(3,4-epoxycyclohexyl)ethyl group as represented by the general formula (I) given above as the reactant (B).

The grafting reaction for the preparation of the silicone-grafted polysaccharide is a well known reaction between the carboxyl groups of the polysaccharide compound and the epoxy groups at one molecular chain end of the diorganopolysiloxane in an equimolar reaction ratio. When the molar amount of the carboxyl groups is in excess over the molar amount of the epoxy groups in the reaction mixture, accordingly, an amount of the carboxyl groups in the polysaccharide reactant are left unreacted in the reaction product after completion of the reaction giving a possibility of subsequent adjustment of the carboxyl content of the silicone-grafted polysaccharide for the purpose of optimization of the silicone-grafted polysaccharide relative to the solubility in a solvent and adhesion thereof to the human skins and hairs treated with the cosmetic composition.

The silicone-grafted polysaccharide, when used as the component (a) in the cosmetic composition of the invention, should have an acid value in the range from 10 to 250 mg KOH/g and the content of the organopolysiloxane moiety therein should be in the range from 1 to 60% by weight. When the acid value of the silicone-grafted polysaccharide is too low, the cosmetic composition is poorly adhesive to the human skins and hairs treated therewith while, when the acid value is too high, the coating film formed from the component has poor water resistance. When the content of the organopolysiloxane moiety in the silicone-grafted polysaccharide is too low, the coating film cannot exhibit good water resistance and lubricity to be imparted by the organopolysiloxane moiety while, when the content thereof is too high, good coating films can hardly be formed from the grafted polysaccharide adversely affecting the sustainability thereof. The organopolysiloxane moiety here implied is the portion represented by the general formula

-(-SiR₂-O-)ₙ-SiR₃,

in which each symbol has the same meaning as defined for the general formula (I).

Examples of the carboxyl-containing polysaccharide compound having solubility in an organic solvent as the reactant (A) include hydroxypropyl methyl cellulose phthalate, hydroxypropyl methyl cellulose acetate succinate, cellulose acetate phthalate, carboxymethyl ethyl cellulose, pullulan acetate phthalate and the like, of which hydroxypropyl methyl cellulose phthalate, hydroxypropyl methyl cellulose acetate succinate and cellulose acetate phthalate are preferable in respect of, in addition to their good availability and inexpensiveness, the safety against human body as is evidenced by their use as an enterosoluble coating agent on various kinds of solid medicament forms authenticated by the pharmacopoeia.

The reactant (B) to be reacted with the above described polysaccharide compound as the reactant (A) is a diorganopolysiloxane terminated at one of the molecular chain ends with a 2-(3,4-epoxycyclohexyl)ethyl group as represented by the general formula (I) given above. The group denoted by R in the general formula is, each independently from the others, a monovalent hydrocarbon group having 1 to 10 carbon atoms exemplified by alkyl groups such as methyl, ethyl, propyl and butyl groups, cycloalkyl groups such as cyclopentyl and cyclohexyl groups, alkenyl groups such as vinyl and allyl groups, aryl groups such as phenyl group and aralkyl groups such as benzyl group as well as halogen-substituted hydrocarbon groups such as chloromethyl and 3,3,3-trifluoropropyl groups.

The subscript n in the general formula is a positive integer in the range from 3 to 200. When the value of n is too small, the compound rarely exhibits the characteristic as a polysiloxane while, when the value of n is too large, the organopolysiloxane has a decreased reactivity with the polysaccharide compound as the reactant (A) necessitating an unduly long reaction time.

Following is a description of the process for the preparation of the silicone-grafted polysaccharide compound which is an essential ingredient in the inventive cosmetic composition.

It is known in the prior art that a silicone-grafted polysaccharide compound can be prepared by reacting a polysaccharide derivative with a conventional organopolysiloxane compound in a solvent. This method, however, is industrially very disadvantageous and is not practically undertaken in recent years excepting for the cases where the product is intended for use in a very specific application because the process is very complicated including the steps of post-reaction treatment for the removal of the unreacted reactants and catalyst by washing, recovery of the solvent, drying of the product and so on.

In the preparation method of the silicone-grafted polysaccharide compound undertaken in the invention, a polysaccharide compound having carboxyl groups as one of the reactants is first dissolved in an organic solvent exemplified by ketone solvents such as acetone, methyl ethyl ketone and cyclohexanone and ether solvents such as dioxane and ethyleneglycol monobutyl ether acetate to give a solution into which the diorganopolysiloxane terminated at one of the molecular chain ends with a 2-(3,4-epoxycyclohexyl)ethyl group is added as the other reactant, referred to as an epoxy-terminated silicone hereinafter, to give a reaction mixture. The amount of the epoxy-terminated silicone in the reaction mixture should be such that the molar amount of the epoxy groups introduced thereby is smaller than the molar amount of the carboxyl groups in the polysaccharide compound. The reaction between the epoxy groups and the carboxyl groups proceeds stoichiometrically when the reaction mixture is heated at a temperature in the range from 60 to 200 °C under agitation. The reaction is completed usually within several hours. The reaction product can be separated from the solvent either by evaporation of the solvent from the reaction mixture or by precipitation with admixture of the reaction mixture with an organic poor solvent for the reaction product such as n-hexane.

Since the silicone-grafted polysaccharide compound as an essential ingredient in the inventive cosmetic composition has unreacted carboxyl groups in the molecule, it is possible to impart the silicone-grafted polysaccharide compound with increased hydrophilicity by neutralizing a part or all of the free carboxyl groups with a basic compound exemplified by alkali metal hydroxides such as sodium hydroxide and potassium hydroxide, ammonia and primary, secondary and tertiary amine compounds such as triethylamine, diisopropylamine, monoethanolamine, triethanolamine, diisopropanolamine, aminomethyl propanol, pyridine, piperidine and morpholine.

The content of the above described silicone-grafted polysaccharide compound as the component (a) in the inventive cosmetic composition is, though depending on the types of the cosmetic compositions, in the range from 0.5 to 30.0% by weight. When the content is too low, the desired effects of improvements of the water-resistance and sustainability cannot be fully obtained while, when the content thereof is too high, an undesirable increase is caused in the viscosity of the cosmetic composition.

The other essential ingredient as the component (b) in the inventive cosmetic composition formulated in combination with the component (a) is an organic compound having at least one aliphatic hydroxyl group in a molecule exemplified by ethyl alcohol, propyl alcohol, ethyleneglycol, ethyleneglycol monoalkyl ethers, diethyleneglycol monoalkyl ethers, polylethyleneglycol, propyleneglycol, dipropyleneglycol, 1,3-butyleneglycol, glycerine, diglycerine, polyglycerine, pentaerithritol, saccharose, lactose, xylitol, sorbitol, mannitol, maltitol, carageenan, agar, guar gum, dexistrin, tragacanth gum, locust bean gum, polyvinyl alcohol, polyoxyethylene-based polymeric compounds, polyoxyethylehe/polyoxypropylene copolymer-based polymeric compounds, hyaluronic acid, chondroitin sulfate, chitin and chitosan. These hydroxyl-containing compounds can be used either singly or as a combination of two kinds or more according to need provided that good miscibility with the component (a) can be ensured.

The content of the above described component (b) in the inventive cosmetic composition is, though depending on the types of the cosmetic compositions, in the range from 0.5 to 99.5% by weight. When the content thereof is too low, an undesirable increase is caused in the viscosity of the cosmetic composition. An excessively high content thereof over 99.5% by weight means that the content of the component (a) cannot be 0.5% by weight or higher.

The balance of the above described components (a) and (b), if any, can be selected from cosmetically admissible materials including oil compounds, water, powders, pigments and surface active agents.

In particular, improvements of the cosmetic composition can be sometimes accomplished by compounding the composition with from 0.5 to 50.0% by weight of an oil compound as the component (c) which can be selected from vegetable- or animal-origin oils and fats as well as semi-synthetic oils derived therefrom.

Examples of the oil compound as the component (c) include avocado oil, linseed oil, almond oil, Chinese wax, perilla oil, olive oil, cacao oil, kapok wax, kaya oil, carnauba wax, cod-liver oil, candelilla wax, beaf tallow, hardened beaf tallow, apricot-kernel oil, whale wax, wheat germ oil, sesame oil, rice germ oil, rice bran oil, sugarcane wax, sasanqua oil, safflower oil, Chinese tung oil, cinnamon oil, jojoba wax, shellac wax, turtle oil, soybean oil, tea kernel oil, camellia oil, corn oil, lard, rapeseed oil, Japanese tung oil, rice bran wax, palm oil, palm kernel oil, castor oil, hardened castor oil, castor oil fatty acid methyl ester, sunflower oil, grape oil, bayberry wax, jojoba oil, macadamia nut oil, beeswax, mink oil, cottonseed oil, cottonseed wax, Japanese wax, montan wax, coconut oil, hardened coconut oil, coconut oil fatty acid triglyceride, mutton tallow, peanut oil, lanolin, liquid lanolin, reduced lanolin, lanolin alcohol, hardened lanolin, lanolin acetate, lanolin fatty acid isopropyl ester, POE-lanolin alcohol ether, POE-lanolin alcohol acetate, polyethyleneglycol lanolin fatty acid ester, POE-hydrogenated lanolin alcohol ether, yolk oil, monobenzylidene sorbitol and dibenzylidene sorbitol as the first class of the oil compounds.

Examples of hydrocarbon oils as a second class of the oil compounds include ozokerite, squalane, squalene, ceresin, paraffin, paraffin wax, liquid paraffin, pristane, polyisobutylene, microcrystalline wax and petrolatum.

Examples of higher fatty acids as a third class of the oil compounds include lauric acid, myristic acid, palmitic acid, stearic acid, behenic acid, undecylenic acid, oleic acid, linoleic acid, arachidonic acid, eicosapentanoic acid, docosahexaenic acid, isostearic acid and 12-hydroxystearic acid.

Examples of higher alcohols as a fourth class of the oil compounds include lauryl alcohol, myristyl alcohol, palmityl alcohol, stearyl alcohol, behenyl alcohol, hexadecyl alcohol, oleyl alcohol, isostearyl alcohol, hexyldodecanol, octyldodecanol, sedostearyl alcohol, 2-decyltetradecynol, cholesterol, phytosterol, POE cholesterol ether, monostearyl glycerin ether and monooleyl glycerin ether.

Examples of ester oils as a fifth class of the oil compounds include diisobutyl adipate, 2-hexyldecyl adipate, di-2-heptylundecyl adipate, N-alkylglycol monoisostearate, isocetyl isostearate, trimethylolpropane triisostearate, ethyleneglycol di-2-ethylhexanoate, neopentylglycol di-2-ethyl hexanoate, cetyl 2-ethylhexanoate, trimethylol propane tri-2-ethyl hexanoate, pentaerithritol tetra-2-ethyl hexanoate, cetyl octanoate, octyldodecyl gum ester, oleyl oleate, octyldodecyl oleate, decyl oleate, neopentylglycol dicaprate, triethyl citrate, 2-ethylhexyl succinate, ethyl acetate, isocetyl stearate, butyl stearate, diisopropyl sebacate, di-2-ethylhexyl sebacate, cetyl lactate, myristyl lactate, isopropyl palmitate, 2-ethylhexyl palmitate, 2-hexyldecyl palmitate, 2-heptylundecyl palmitate, cholesterol 12-hydroxystearate, dipentaerithritol fatty acid esters, isopropyl myristate, 2-octyldecyl myristate, 2-hexyldecyl myristate, myristyl myristate, hexyldecyl dimethyloctanoate, ethyl laurate, hexyl laurate, 2-octyldodecyl N-lauroyl-L-glutamate, diisostearyl malate, dextrin palmitic acid ester, dextrin stearic acid esters, dextrin 2-ethylhexanoic acid palmitic acid mixed ester, saccharose palmitic acid ester and saccharose stearic acid esters.

Examples of glyceride oils as a sixth class of the oil compounds include acetoglyceryl, glyceryl diisooctanoate, glyceryl triisostearate, glyceryl triisopalmitate, glyceryl tri-2-ethylhexanoate, glyceryl monostearate, glyceryl di-2-heptylundecanoate and glyceryl trimyristate.

Examples of silicone oils as a seventh class of the oil compounds include dimethylpolysiloxane oils, methylphenylpolysiloxane oils, methylhydrogenpolysiloxane oils, octamethyl cyclotetrasiloxane, decamethyl cyclopentasiloxane, dodecamethyl cyclohexasiloxane, tetrahydrotetramethyl cyclotetrasiloxane, higher alcohol-modified silicone oils such as stearoxy silicone oils, higher fatty acid-modified silicone oils, fluorine-modified silicone oils, amino-modified silicone oils, alklyl-modified silicone oils, higher fatty acid ester-modified silicone oils, silicone resins and silicone rubbers.

Examples of fluorocarbon oils as an eighth class of the oil compounds include perfluoro polyethers, perfluoro decahydronaphthalene and perfluorooctane.

The above described various kinds of oil compounds can be used either singly or as a combination of two kinds or more, if compatible. The content of the oil compounds as the component (c) in the inventive cosmetic composition is, if added, in the range from 0.5 to 50.0% by weight.

The cosmetic composition of the invention, of which the essential ingredients are the components (a) and (b), may contain water or can be a water-base cosmetic composition. The content of water as the component (d) in this case is in the range from 0.5 to 99.0% by weight provided that good solubility of the components (a) and (b) can be ensured.

While the cosmetic composition of the invention can be formulated with the components (a) and (b) alone optionally in combination with an oil compound as the component (c) and/or water as the component (d), it is further optional that the cosmetic composition comprises a powder or pigment as the component (e), surface active agent as the component (f), crosslinked organopolysiloxane as the component (g) and/or silicone resin as the component (h).

Examples of inorganic powders as a first class of the component (e) include titanium dioxide, zirconium oxide, zinc oxide, cerium oxide, magnesium oxide, barium sulfate, calcium sulfate, magnesium sulfate, calcium carbonate, magnesium carbonate, talc, mica, kaolin, sericite, muscovite, synthetic mica, phlogopite, lepidolite, biotite, lithia mica, silicic acid, silica, aluminum silicate, magnesium silicate, aluminum magnesium silicate, calcium silicate, barium silicate, strontium silicate, metal tungstates, hydroxyapatite, vermiculite, heidilite, bentonite, montmorillonite, hectolite, zeolite, ceramic powders, calcium monohydrogenphosphate, alumina, aluminum hydroxide and boron nitride.

Examples of organic powders as a second class of the component (e) include powders of polyamide resin, polyester resin, polyethylene, polypropylene, polystyrene, polyurethane resin, benzoguanamine resin, polymethylbenzoguanamine resin, polytetrafluoroethylene resin, polymethyl methacrylate resin, cellulose, silk, nylon resin, 12-nylon resin, 6-nylon resin, styrene/acrylic acid-copolymeric resin, divinylbenzene/styrene- copolymeric resin, polyvinyl chloride resin, urea resin, phenolic resin, fluorocarbon resin, silicone resin, acrylic resin, melamine resin, epoxy resin, polycarbonate resin, microcrystalline cellulose, starch, lauroyl lysine and metal soaps such as zinc stearate, aluminum stearate, calcium stearate, magnesium stearate, zinc myristate, magnesium myristate, zinc cetylphosphate, calcium cetylphosphate and zinc sodium cetylphosphate.

Examples of pigments as a third class of the component (e) include inorganic red pigments such as iron oxide, iron hydroxide and iron titanate, inorganic brown pigments such as γ-iron oxide, inorganic yellow pigments such as yellow iron oxide and Chinese yellow, inorganic black pigments such as black iron oxide and carbon black, inorganic violet pigments such as manganese violet and cobalt violet, inorganic green pigments such as chromium hydroxide, chromium oxide and cobalt titanate, inorganic blue pigments such as Prussian blue and ultramarine, tar-based dyes converted into a lake, natural dyes converted into a lake, composites of these powders, pearlescent pigments such as titanium dioxide-coated mica, bismuth oxychloride, titanium dioxide-coated bismuth oxychloride, titanium dioxide-coated talc, fish scale flakes and titanium dioxide-coated colored micas and tar dyes such as Red #3, Red #104, Red #106, Red #201, Red #202, Red #204, Red #205, Red #220, Red #226, Red #227, Red #228, Red #230, Red #401, Red #505, Yellow #4, Yellow #5, Yellow #202, Yellow #204, Yellow #5, Yellow #401, Blue #1, Blue #2, Blue #201, Blue #404, Green #3, Green #201, Green #204, Green #205, Orange #201, Orange #203, Orange #204, Orange #206 and Orange #207 and natural dyes such as carminic acid, laccaic acid, brazilin and crocin.

These particulate materials and pigments are not particularly limitative in the particle configuration including spherical or globular particles, needle-formed particles and platelet particles and also not limitative in the particle size. Composites of two or more kinds thereof can be used. It is optional that the powder is used after a surface treatment with an oil agent, silicone oil and fluorocarbon compound.

Various kinds of surface active agents can be used as the component (f) in the inventive cosmetic composition. Examples of suitable surface active agents include carboxylic acid salts such as saturated and unsaturated fatty acid soaps, e.g., sodium stearate and triethanolamine oleate, alkyl ether carboxylic acids and salts thereof and condensation products of an amino acid and fatty acid, amido ether carboxylic acid salts, α-sulfo fatty acid ester salts, α-acylsulfonic acids, alkylsulfonic acids, alkenesulfonic acid salts, sulfonic acid salts of fatty acid esters, sulfonic acid salts of fatty acid amides, sulfonic acid salts of a formalin condensation product with an alkylsulfonic acid salt, alkyl sulfate esters, secondary alcohol sulfate esters, alkyl and aryl ether sulfate esters, sulfate esters of fatty acid esters and sulfate esters of fatty acid alkylolamide as the sulfate esters, alkyl phosphates, alkenyl phosphates, ether phosphates, alkylaryl ether phosphates, alkylamide phosphates and N-acylamino acid-based active agents, cationic surface active agents exemplified by amine salts such as alkylamine salts, polyamine and aminoalcohol fatty acid derivatives, alkyl quaternary ammonium salts, aromatic quaternary ammonium salts, pyridinium salts and imidazolinium salts, non-ionic surface active agents exemplified by sorbitan fatty acid esters, glycerin fatty acid esters, polyglycerin fatty acid esters, propyleneglycol fatty acid esters, polyethyleneglycol fatty acid esters, saccharose fatty acid esters, polyoxyethylene alkyl ethers, polyoxypropylene alkyl ethers, polyoxyethylene alkylphenyl ethers, polyoxyethylene fatty acid esters, polyoxyethylene sorbitan fatty acid esters, polyoxyethylene glycerin fatty acid esters, polyoxyethylene propyleneglycol fatty acid esters, polyoxyethylene castor oils, polyoxyethylene hardened castor oils, polyoxyethylene phytostannol ethers, polyoxyethylene phytosterol ethers, polyoxyethylene cholesteryl ethers, polyoxyalkylene-modified organopolysiloxanes, polyoxyalkylene/alkyl-comodified organopolysiloxanes, polyoxyalkylene/fluoroalkyl-comodified organopolysiloxanes, polyoxyalkylene-organopolysiloxane block copolymers, alkanolamides, sugar ethers and sugar amides, and amphoteric surface active agents exemplified by betaine compounds, aminocarboxylates and imidazoline derivatives.

Further classes of optional additives include crosslinked organopolysiloxanes as the component (g) and silicone resins such as acrylic/silicone graft or block copolymers and three-dimensional silicones as the component (h).

Actual application types of the inventive cosmetic compositions include skincare compositions, hair-care treatment compositions, antiperspirant compositions, makeup compositions, anti-ultraviolet compositions, nail polish compositions and so on without particular limitations. The preparation forms of the inventive cosmetic compositions can be solutions, milky lotions, creams, solids, pastes, gels, powders, laminates, mousses and aerosol sprays.

In the following, the present invention is described in more detail by way of examples for the synthetic preparation procedure of the silicone-grafted polysaccharide compounds and formulations of several cosmetic compositions containing the silicone-grafted polysaccharide compound.

### Synthetic Preparation 1.

Into a glass flask equipped with a stirrer, thermometer and reflux condenser were introduced 75 g of a hydroxypropyl methyl cellulose phthalate (HPMCP) containing 33.4% by weight of benzoyl groups (HP-55, a product by Shin-Etsu Chemical Co.) to give 168.2 mmoles of carboxyl groups, 25 g of an epoxy-terminated dimethylpolysiloxane expressed by the formula

E-CH₂CH₂-(-SiMe₂-O-)₂₈-SiMe₃,

In which E is a 3,4-epoxycyclohexyl group and Me is a methyl group, containing 11.0 mmoles of epoxy groups corresponding to an epoxy equivalent of 2.270 g/mole and 400 g of cyclohexanone to give a uniform reaction mixture which was heated with agitation at 150 °C for 5 hours under a stream of nitrogen gas to give a slightly cloudy, light yellow liquid.

The reaction mixture obtained above was cooled down to 30 °C and admixed under agitation with 1 liter of n-hexane to precipitate a polymeric product which was collected by filtration and vacuum-dried at 100 °C for 3 hours to give 92 g of a light yellow powder as a dried product having an acid value of 88.5 mg KOH/g.

An infrared absorption spectrophotometric analysis was undertaken for this powder product to give absorption bands originating in dimethylpolysiloxane at 1262 cm⁻¹ assignable to Si-CH₃, at 1071 cm⁻¹ and 1123 cm⁻¹ assignable to Si-O-Si and at 801 cm⁻¹ assignable to (CH₃)₂-Si-O, in addition to the characteristic absorption bands originating in the hydroxypropyl methyl cellulose phthalate.

Further, the product was subjected to quantitative determination of the carboxybenzoyl groups by the method specified in Japanese Pharmacopoeia for HPMCP to give a value of 23.5% by weight indicating that the reaction was substantially quantitative. The content of the organopolysiloxane moiety in the product was 24.5% by weight as calculated from the yield of the product.

### Synthetic Preparation 2.

The synthetic procedure was substantially the same as in Synthetic Preparation 1 described above except that the amount of the hydroxypropyl methyl cellulose phthalate was decreased from 75 g to 50 g to give 112.1 mmoles of carboxyl groups and the amount of the epoxy-terminated dimethylpolysiloxane was increased from 25 g to 50 g to give 22.0 mmoles of the epoxy groups. The yield of the light yellow powder product was 91 g, which had an acid value of 51.2 mg KOH/g.

An infrared absorption spectrophotometric analysis was undertaken for this powder product to give absorption bands originating in dimethylpolysiloxane at 1264 cm⁻¹ assignable to Si-CH₃, at 1073 cm⁻¹ and 1125 cm⁻¹ assignable to Si-O-Si and at 801 cm⁻¹ assignable to (CH₃)₂-Si-O, in addition to the characteristic absorption bands originating in the hydroxypropyl methyl cellulose phthalate.

Further, the product was subjected to quantitative determination of the carboxybenzoyl groups by the method specified in Japanese Pharmacopoeia for HPMCP to give a value of 13.6% by weight indicating that the reaction was substantially quantitative. The content of the organopolysiloxane moiety in the product was 49.5% by weight as calculated from the yield of the product.

### Synthetic Preparation 3.

The synthetic procedure was substantially the same as in Synthetic Preparation 1 described above except that the hydroxypropyl methyl cellulose phthalate was replaced with the same amount of a hydroxypropyl methyl cellulose acetate succinate (AS-LG, a product by Shin-Etsu Chemical Co.) containing 15.6% by weight of the succinoyl groups to give 115.6 mmoles of carboxyl groups. The yield of the light yellow powder product was 93 g, which had an acid value of 60.0 mg KOH/g.

An infrared absorption spectrophotometric analysis was undertaken for this powder product to give absorption bands originating in dimethylpolysiloxane at 1238 cm⁻¹ assignable to Si-CH₃, at 1057 cm⁻¹ and 1122 cm⁻¹ assignable to Si-O-Si and at 803 cm⁻¹ assignable to (CH₃)₂-Si-O, in addition to the characteristic absorption bands originating in the hydroxypropyl methyl cellulose acetate succinate.

Further, the product was subjected to quantitative determination of the succinoyl groups to give a value of 10.8% by weight indicating that the reaction was substantially quantitative. The content of the organopolysiloxane moiety in the product was 23.8% by weight as calculated from the yield of the product.

### Synthetic Preparation 4.

A partial neutralization product of the silicone-grafted polysaccharide compound obtained in Synthetic Preparation 1 was prepared in the following manner. Thus, 50 parts by weight of the light yellow powder product of Synthetic Preparation 1 were dissolved in 50 parts by weight of a 1:9 by weight mixture of water and ethyl alcohol and the solution was admixed with 4.5 parts by weight of a 50% aqueous solution of potassium hydroxide under agitation so that 50% of the free carboxyl groups in the starting silicone-grafted HPMCP were neutralized.

### Synthetic Preparation 5.

The same partial neutralization procedure as in Synthetic Preparation 4 was undertaken excepting for the replacement of 4.5 parts by weight of the 50% aqueous potassium hydroxide solution with 3.5 parts by weight of 2-amino-2-methyl-1-propanol to neutralize 50% of the carboxyl groups in the starting graft polymer.

### Synthetic Preparation 6.

A 50% neutralization product of the silicone-grafted polysaccharide compound obtained in Synthetic Preparation 2 was prepared in the following manner. Thus, 50 parts by weight of the light yellow powder product of Synthetic Preparation 2 were dissolved in 50 parts by weight of a 1:9 by weight mixture of water and ethyl alcohol and the solution was admixed with 2.5 parts by weight of a 50% aqueous solution of potassium hydroxide under agitation so that 50% of the free carboxyl groups in the starting silicone-grafted HPMCP were neutralized.

### Example 1.

A skincare cream composition was prepared in the following formulation.

| Ingredient | % by weight |
|---|---|
| (1) Stearic acid | 2.0 |
| (2) Cetanol | 1.0 |
| (3) Squalane | 10.0 |
| (4) Stearic acid monoglyceride | 2.0 |
| (5) Jojoba oil | 5.0 |
| (6) Olive oil | 5.0 |
| (7) Dimethylsilicone oil, 200 cS | 5.0 |
| (8) Sorbitan sesquioleate | 1.0 |
| (9) Polyoxyethylene sorbitan monooleate | 2.0 |
| (10) 1,3-Butyleneglycol | 7.0 |
| (11) Silicone-grafted polysaccharide ¹⁾ | 7.0 |
| (12) Triethanolamine | 1.0 |
| (13) Purified water | balance |

| | |
|---|---|
| 1) Synthetic Preparation 1 | |

The steps of the preparation procedure were as follows.
A: Ingredients (1) to (8) were melted together with heating.
B; Ingredients (9), (11) and (12) were dissolved together in (10).
C: The mixture A was admixed with the solution B to effect emulsification.
D: Emulsified mixture C was cooled to room temperature.

The thus prepared skincare cream composition was subjected to an organoleptic evaluation test by 20 female expert panel members for six testing items including: (1) smoothness in application to their skin; (2) non-stickiness of the treated skin; (3) smoothness of the treated skin surface; (4) refreshingness of the treated skin; (5) sustainability of the coating film; and (6) overall evaluation.

Each of the panel members was requested to give an evaluation point of 1 to 5 for each of these testing items under the criteria of: 5 points (excellent), 4 points (good), 3 points (fair), 2 points (somewhat poor) and 1 point (poor) and the evaluation points given by the 20 panel members were averaged for each testing item to give the results in four ratings of A, B, C and D as shown in Table 1 below in which rating A was given for at least 4.0 of the averaged evaluation points, B was given for at least 3.0 of the averaged evaluation points, C was given for at least 2.0 of the averaged evaluation points and D was given when the averaged evaluation point was smaller than 2.0.

The resukts of the thus performed organoleptic evaluation tests are shown in Table 1 below.

### Comparative Example 1.

The formulation and preparation procedure for a skincare cream composition were substantially the same as in Example 1 excepting for the omission of the silicone-grafted polysaccharide compound and a corresponding increase in the amount of the purified water.

The results of the organoleptic evaluation tests of this comparative cream composition are shown in Table 1 below.

**Table 1**

| Testing Item | Example 1 | Comparative Example 1 |
|---|---|---|
| (1) | B | B |
| (2) | A | C |
| (3) | A | B |
| (4) | A | C |
| (5) | A | D |
| (6) | A | C |

### Example 2.

A cream rinse composition was prepared in the following formulation.

| Ingredient | % by weight |
|---|---|
| (1) Alkyl trimethylammonium chloride | 2.0 |
| (2) Cetanol | 3.0 |
| (3) Octyl palmitate | 1.0 |
| (4) Crosslinked organopolysiloxane ²⁾ | 2.0 |
| (5) Propyleneglycol | 5.0 |
| (6) Silicone-grafted polysaccharide ³⁾ | 2.0 |
| (7) Purified water | balance |

| | |
|---|---|
| 2) KSG 16, a product by Shin-Etsu Chemical Co. | |
| 3) Synthetic Preparation 5 | |

The steps of the preparation procedure were as follows.
A: Ingredients (1) and (5) to (7) were melted together by heating.
B: Ingredients (2) to (4) were melted together by heating and the mixture was added to A.
C: The mixture B was cooled to room temperature.

The thus prepared cream rinse composition was subjected to an organoleptic evaluation test by 20 female expert panel members for 5 testing items including: (1) smoothness in application; (2) smoothness or lubricity of hair after drying; (3) dry and non-sticky touch of treated hair; (4) refreshing feeling; and (5) overall evaluation.

The results of the organoleptic evaluation tests of this cream rinse composition reported under the same criteria as in Example 1 are shown in Table 2 below.

### Comparative Example 2.

The formulation and preparation procedure for a cream rinse composition were substantially the same as in Example 2 excepting for the omission of the silicone-grafted polysaccharide compound and a corresponding increase in the amount of the purified water.

The results of the organoleptic evaluation tests of this comparative cream rinse composition are shown in Table 2 below.

**Table 2**

| Testing Item | Example 2 | Comparative Example 2 |
|---|---|---|
| (1) | B | B |
| (2) | A | B |
| (3) | A | B |
| (4) | A | B |
| (5) | A | B |

### Example 3.

An anti-perspirant composition was prepared in the following formulation.

| Ingredient | % by weight |
|---|---|
| (1) Polyoxyethylene organopolysiloxane ⁴⁾ | 0.5 |
| (2) Aluminum chlorohydroxide | 3.0 |
| (3) Talc | 5.0 |
| (4) Silicone-grafted polysaccharide ⁵⁾ | 10.0 |
| (5) Ethyl alcohol | 15.0 |
| (6) Hydroxyethylcellulose | 2.0 |
| (7) Purified water | balance |

| | |
|---|---|
| 4) KF 6011, a product by Shin-Etsu Chemical Co. | |
| 5) Synthetic Preparation 4 | |

The steps of the preparation procedure were as follows.
A: Ingredients (1) to (7) were mixed together.
B: A roll-on cartridge was filled with the mixture A.

### Example 4.

A cream foundation composition was prepared in the following formulation.

| Ingredient | % by weight |
|---|---|
| (1) Crosslinked dimethylpolysiloxane ⁶⁾ | 7.0 |
| (2) Glycerin trioctanoate | 3.0 |
| (3) Dimethyl silicone oil, 6 cS | 8.0 |
| (4) Silicone powder ⁷⁾ | 1.0 |
| (5) Sodium chloride | 0.5 |
| (6) 1,3-Butyleneglycol | 7.0 |
| (7) Ethyl alcohol | 4.0 |
| (8) Silicone-grafted polysaccharide ⁸⁾ | 16.0 |
| (9) Pigments | q.s. |
| (10) Silicone-grafted acrylic polymer ⁹⁾ | 5.0 |
| (11) Purified water | balance |

| | |
|---|---|
| 6) KSG 21, a product by Shin-Etsu Chemical Co. | |
| 7) KSP 100, a product by Shin-Etsu Chemical Co. | |
| 8) Synthetic Preparation 6 | |
| 9) KP 545, a product by Shin-Etsu Chemical Co. | |

The steps of the preparation procedure were as follows.
A: Ingredients (1) to (4) were mixed together.
B: Ingredients (5) to (8) and (11) as a mixture were added to the mixture A.
C: Ingredients (9) and (10) as a mixture were added to the mixture B.

The thus prepared anti-perspirant composition could be applied to the skin without stickiness and with good spreadability and exhibited excellent non-stickiness and refreshingness of the treated skin and sustainability of cosmetic finish.

### Example 5.

An anti-ultraviolet cream composition was prepared in the following formulation.

| Ingredient | % by weight |
|---|---|
| (1) Fine powder of silicone-treated zinc oxide | 10.0 |
| (2) Fine powder of silicone-treated titanium dioxide | 10.0 |
| (3) Trimethylsiloxy silicate ¹⁰⁾ | 8.0 |
| (4) Decamethyl cyclopentasiloxane | 20.0 |
| (5) Glycerin trioctanoate | 5.0 |
| (6) Corsslinked methylpolysiloxane ¹¹⁾ | 5.0 |
| (7) Polyoxyethylene/alkyl-comodified silicone ¹²⁾ | 1.0 |
| (8) Octyl 4-methoxycinnamate | 5.0 |
| (9) Silicone-grafted polysaccharide ¹³⁾ | 15.0 |
| (10) Sodium chloride | 0.5 |
| (11) Ethyl alcohol | 5.0 |
| (12) Purified water | balance |

| | |
|---|---|
| 10) KF 7312J, a product by Shin-Etsu Chemical Co. | |
| 11) KSG 21, a product by Shin-Etsu Chemical Co. | |
| 12) KF 6026, a product by Shin-Etsu Chemical Co. | |
| 13) Synthetic Preparation 4 | |

The steps of the preparation procedure were as follows.
A: Ingredients (5) to (8) were mixed with a 1/4 portion of (4).
B:; Ingredients (9) to (12) as a mixture were added to the mixture A.
C: Ingredients (1) to (3) and remainder of (4) as a mixture were added to the mixture A.
D: Mixture C was added to B.

The thus prepared anti-ultraviolet cream composition could be applied to the skin without stickiness and with goof spreadability and exhibited excellent non-stickiness and refreshingness of the treated skin and sustainability of cosmetic finish.

### Example 6.

A nail polish composition was prepared in the following formulation.

| Ingredient | % by weight |
|---|---|
| (1) Silicone-grafted polysaccharide ¹⁴⁾ | 20.0 |
| (2) Propyleneglycol | 3.0 |
| (3) Ethyl alcohol | 70.0 |
| (4) Pigments, hydrophilized with POE | q.s. |
| (5) Purified water | balance |

| | |
|---|---|
| 14) Synthetic Preparation 5 | |

The nail polish composition was prepared by dispersing the pigments in the mixture of the other ingredients. The thus prepared nail polish composition could exhibit excellent glossiness without using an aromatic or ester solvent such as toluene, ethyl acetate and butyl acetate giving a coating film of high hardness.

### Example 7.

A hair setting composition was prepared in the following formulation.

| Ingredient | % by weight |
|---|---|
| (1) Silicone-grafted polysaccharide ¹⁵⁾ | 10.0 |
| (2) 1,3-Butyleneglycol | 2.0 |
| (3) Ethyl alcohol | 80.0 |
| (4) Purified water | balance |

| | |
|---|---|
| 15) Synthetic Preparation 5 | |

A hair setting composition was prepared by blending the above listed ingredients and an aerosol spray can was filled with the composition together with a propellant therefor. The hair setting composition was found excellent in respect of sustainability of the hair-setting effect as well as in respect of the glossiness of the treated hair and touch feeling thereof.

## Claims

1. A silicone-grafted polysaccharide compound which is a reaction product between (A) a polysaccharide compound having at least one carboxyl group in a molecule and having solubility in an organic solvent and (B) a diorganopolysiloxane represented by the general formula
E-CH₂CH₂-(-SiR₂-O-)ₙ-SiR₃,
in which E is a 3,4-epoxycyclohexyl group, each R is, independently from the others, a monovalent hydrocarbon group having 1 to 10 carbon atoms and the subscript n is a positive integer in the range from 3 to 200, having an acid value in the range from 10 to 250 mg KOH/g and containg from 1 to 60% by weight of the diorganopolysiloxane moiety.

2. The silicone-grafted polysaccharide compound as claimed in claim 1 in which the polysaccharide compound as the reactant (A) is selected from the group consisting of hydroxypropyl methyl cellulose phthalate, hydroxypropyl methyl cellulose acetate succinate, cellulose acetate phthalate, carboxymethyl ethyl cellulose and pullulan acetate.

3. A cosmetic composition which comprises, as a blend:
(a) from 0.5 to 30.0% by weight of the silicone-grafted polysaccharide compound defined in claim 1; and
(b) from 0.5 to 99.5% by weight of an organic compound having, in a molecule, at least one aliphatic hydroxyl group,
the balance, if any, being compounds admissible in a cosmetic composition.

4. The cosmetic composition as claimed in claim 3 in which the balance to the components (a) and (b) comprises;
(c) from 0.5 to 50.0% by weight of an oil compound.

5. The cosmetic composition as claimed in claim 3 in which the balance to the components (a) and (b) comprises;
(d) from 0.5 to 99.0% by weight of water.

6. The cosmetic composition as claimed in claim 3 in which at least a part of the carboxyl groups in the silicone-grafted polysaccharide compound as the component (a) are in the form of a salt formed by neutralization with a basic compound.

7. The cosmetic composition as claimed in claim 3 in which the organic compound as the component (b) is an aliphatic alcohol compound at least partly soluble in water.

8. The cosmetic composition as claimed in claim 3 in which the organic compound as the component (b) is a polymeric compound having hydroxyl groups and soluble in water.

9. The cosmetic composition as claimed in claim 4 in which the oil compound as the component (c) is in the form of liquid at room temperature.

10. The cosmetic composition as claimed in claim 4 in which the oil compound as the component (c) is a silicone oil.

11. The cosmetic composition as claimed in claim 3 in which the balance to the components (a) and (b) comprises:
(e) a particulate material.

12. The cosmetic composition as claimed in claim 3 in which the balance to the components (a) and (b) comprises:
(f) a surface active agent.

13. The cosmetic composition as claimed in claim 3 in which the balance to the components (a) and (b) comprises:
(g) a crosslinked organopolysiloxane.

14. The cosmetic composition as claimed in claim 3 in which the balance to the components (a) and (b) comprises:
(h) a graft copolymer or block copolymer consisting of an acrylic moiety and organopolysiloxane moiety.
